Europäisches Patentamt

European Patent Office

Office européen des brevets

(11) Publication number: **0 364 158**
**A1**

## (12) EUROPEAN PATENT APPLICATION

(21) Application number: 89310200.4

(22) Date of filing: 05.10.89

(51) Int. Cl.⁵: **A61B 5/00 , A61B 5/16 , A61B 5/103**

The title of the invention has been amended (Guidelines for Examination in the EPO, A-III, 7.3).

(30) Priority: 11.10.88 GB 8823765

(43) Date of publication of application:
18.04.90 Bulletin 90/16

(84) Designated Contracting States:
AT BE CH DE ES FR GB GR IT LI LU NL SE

(71) Applicant: **ICI AMERICAS INC.**
**Concord Pike & New Murphy Road**
**Wilmington Delaware 19897(US)**

(72) Inventor: **Laudadio, Charles**
**117 Parrish Lane**
**Wilmington, DE(US)**

(74) Representative: **Smith, Stephen Collyer et al**
**Imperial Chemical Industries PLC Legal**
**Department: Patents Po Box 6 Bessemer**
**Road**
**Welwyn Garden City Herts, AL7 1HD(GB)**

(54) Tactile discrimination device and its use in assessing neurological function.

(57) A device for assessing the degree of neurological dysfunction associated with diseases such as diabetes may be useful in the intermediate diagnosis of such a disease. An individual's ability to discriminate is investigated by successive application of calibrated probes (0...7; 11;21;31) of different cross-sections to the skin.

*Fig.1.*

## METHOD AND DEVICE

The invention is directed to an intermediate method for assessing human neurological function and/or detecting human neurological dysfunction. In particular, it is directed to a procedure which involves pressing against the subject's skin a series of probes varying in cross-section and obtaining a discriminatory response with regard to the relative cross-sectional size of each probe. The magnitude of this response is then compared with a normal response as determined from a population of subjects having no neurological dysfunction. The invention is also directed to an instrument having a series of probes of varying cross-section which is used in the procedure.

Recent developments in the field of quantitative sensory testing have documented the utility and value of such procedures for the early detection of neurological dysfunction which often accompanies diseases such as diabetes. The fact that the severity of neurological dysfunction is proportional to the progress of the underlying disease has been established by quantitative sensory testing employing relatively expensive instruments and time consuming procedures. While such procedures and equipment are well appreciated, there remains a need for a non-invasive, less time consuming procedure for longitudinal monitoring of "at risk" patients as well as a quick method for screening and detecting early states of neuropathy.

The present invention provides a simple test and instrument which can be used for rapid testing (approximately 2 minutes per site) when conducted by a trained technician. The procedure is sufficiently accurate to distinguish between normal subjects, subjects with subclinical neuropathy, and subjects with clear clinical evidence of neuropathy.

It is an object therefore to provide a method and instrument for use in determining the severity of neurological dysfunction by measuring the subject's ability to discriminate the difference in calibrated cross-sectional dimensions (for example the diameters) of various size probes when applied to the skin with a calibrated instrument. It is another object of the invention to provide a tactile circumference discriminator instrument which can be used to provide a cross-sectional stimulus of known dimension.

In general, the method is a psychophysiological measurement of a subject's ability to integrate information from distal neuroreceptors in order to differentiate cross-sectional area stimuli. This measurement is determined by sequentially pressing two flat probe surfaces having larger and smaller cross-sectional measurements against the surface of the subject's skin using relatively identical pressures and determining if the subject can distinguish the dimensional difference between the probe surfaces. Normal subjects can discriminate at the fingertip between cross sections having about 0.8-1 mm differences while those having severe neurological dysfunction generally cannot distinguish a difference of about 2.8 mm. Therefore, the method provides for a fast, non-invasive intermediate diagnostic procedure for screening subjects for neurological dysfunction as a means for identifying subjects with subclinical neuropathy and those having clear clinical evidence of neuropathy, as well as other related disorders.

The invention involves an intermediate diagnostic method and instrument for detecting human neurological dysfunction which comprises the procedure of pressing against an area of the subject's skin hidden from the subject's view a series of three or more large to small probes having graduated cross-sectional dimension ranging in size from about 1 mm -9 mm in cross-section to gain a discriminatory response from said subject with regard to the relative cross-sectional end surface size of said probes when pressed against an area of the skin in sequential pairs, and thereafter comparing said response with a normal response representing no dysfunction. The invention is also directed to an instrument comprising a central base member which holds a series of probes of differing cross-sectional dimensions ranging from about 1 mm to about 9 mm. The probes are preferably cylindrical rods having flat end surfaces having graduated diameters of from about 1 to 9 mm.

The surface of the skin to be tested is selected at a distal location wherein the nerve receptor density is highest. Preferred such locations are toe pads and finger tips. It is important to first note whether the surface of the skin is damaged, unclean or callused. If they appear to be such, it is best to select another location for testing. When possible, the test should be carried out at the tip of the large toe point because it is this location at which distal neuropathy normally occurs earliest. The reliability of the test tends to improve if the skin is at normal temperature. If the temperature of the skin surface appears to be cold, circulation in the area should be increased until the temperature at the surface is stabilized between 31-37° C for the upper limbs and 30-36° C for the lower limbs and should be held there for the entire test.

The objective in the test is to determine whether the subject can detect the difference between a large and a small cross-sectional probe pressed against the surface under examination when shielded or held from view. This can be accomplished by asking the subject to look in a different direction, to close his/her eyes, or by holding the subject's body portion being tested behind a screen.

An effort is made to maintain a relatively constant pressure not exceeding about 5 grams per square centimeter so that the deep receptors below the skin surface are not stimulated. Therefore, in order to maintain a relatively constant pressure, the force is reduced as the test proceeds from the larger cross-sectioned probes through the smaller cross sectioned probe. As a general rule, the amount of pressure just required to induce the blush point at the finger or toenail is satisfactory.

The cross-sectional shape of the probe placed in contact with the surface of the skin may be any shape such as square, oval, circular, X-shaped, star shaped, either solid or hollow centered. Usually, it is preferred to conduct the test with probes having the same overall cross-sectional shape. Most preferred are those shapes wherein the cross-sectional surface area is held constant by employing a hollow center.

The material of construction for the probes may be either thermally conducting such as metal, glass and ceramic or non-conducting such as in plastics, hard rubber or wood.

The size of the probe surface to be placed against the surface of the skin may range in cross-sectional diameter from about 1 mm to about 9 mm. The graduated difference in probe-to-probe cross-sectional size preferably ranges from 0.2 mm to about 0.4 mm and most preferably is an average of about 0.3 mm. While larger and smaller cross sections can be used, there appears to be no relative advantage in doing so.

In applying the test, a modified two-alternative forced choice procedure is utilized, which means that when a pair of probes is held against the surface of the skin in sequence, the subject is required to determine which of the two probes is larger in diameter (or other cross sectional dimension). If the subject cannot distinguish one over the other, he or she must guess. The subject is tested with pairs of different size probes according to a predetermined set procedure until the subject has made two errors at a given combination. Normally, the test is started by pressing the largest and smallest probe against the skin surface. If the subject cannot discriminate between the probes the test is terminated. If the subject can correctly discriminate, the test then continues by touching the smallest and next largest and so on according to a predetermined sequence which involves gradually working toward the medium size probe until the subject cannot discriminate a pair. This pair difference is then compared with a normal discriminatory response for subjects having no neurological dysfunction for the particular shaped probe employed in the test.

The normal response for those subjects having no neurological dysfunction will vary with the human population sampled. Slight variations can occur depending upon such factors as geographic location, hereditary factors, the physical labor involved in the performance of their profession, dress code and the like. For example, those populations who do not normally wear shoes would most likely provide more uniform response if tested on their index fingers while those populations who normally wear shoes but are involved in a high intensity of hand labor would most likely provide a more uniform response if tested on their large toe.

In establishing a threshold value for the normal response, a population sample must be selected from those known to have no dysfunction as measured by alternative known physiological test procedures.

In designating a normal response, any code which can be used to characterize the subject's ability to discriminate between larger and small cross-sectional surfaces in contact with the skin may be employed. The code need only be known to the tester since the subject being tested need only respond with a "yes" or "no", reply. For example, a normal response may be characterized as a percentage of total correct answers given over an entire size range of probes employed or may be characterized as the ability to distinguish in terms of millimeters the different probe pairs employed over the entire range or their ability to distinguish the difference between probe diameters within a narrow range of diameters. An arbitrary progressive numbering system may be used to identify each probe size which has been previously calibrated for use in the test.

When the test is used as a screening procedure for any given population, the response obtained from any given subject may be compared with the normal response for that population and, if falling outside the normal response, may be judged semiquantitatively to be a candidate for further evaluation by other physiological test means.

The normal response for any population group is based upon the analysis, interpretation and presentation of the numerical data and the size of the group tested. Usually the best value for a normal response will be accurate for 90-95% of those tested. There fore, the diagnostic technique and method employed by the invention is considered to be an intermediate indication for those subjects giving a response different from a statistical norm.

For solid metal probes having a circular shape, those subjects having essentially no dysfunction are able with their finger to differentiate a difference in diameter of 0.8 - 1 mm when the diameter of the smaller probe ranges from about 1.6 - 2.6 mm, and are able to differentiate a diameter of 1.2 -2 mm with the toe pad. However, those who are able to distinguish only greater differences in diameter at either location are considered to be candidates for further testing for disorders related to neurological dysfunction.

In carrying out the diagnostic procedure of the invention, the probes employed in making the sensitivity determination can range from a simple series of solid metal rods ranging in diameter from about 1 mm - 9 mm of about at least 1.0 cm in length which can be hand held individually when pressed against the surface of the skin by a technician. However, the diagnostic procedure may be machine administered wherein the fingers or toes are placed over a small opening in an apparatus through which the probes rise in contact with the surface of the skin according to a program to which the subject responds positively or negatively by pressing a response button which is recorded and analyzed by a computer which calculates the threshold level obtained. An intermediate level instrument for use by a trained technician is found in a hand-held device which holds a series of at least 3 probes ranging in size from 1-9 mm and greater.

The invention is further explained and exemplified with reference to the drawings:

Figure 1 is a top view of an instrument according to the invention.

Figure 1a is a side view of the Figure 1 instrument.

Figure 2 is a sectional view of an alternate probe arrangement held in a coil spring loaded well.

Figure 3 is an alternate hollow probe arrangement mounted in a coil spring loaded barrel.

Figure 4 is a sectional view of an alternate probe arrangement held in an elastomer foam loaded well.

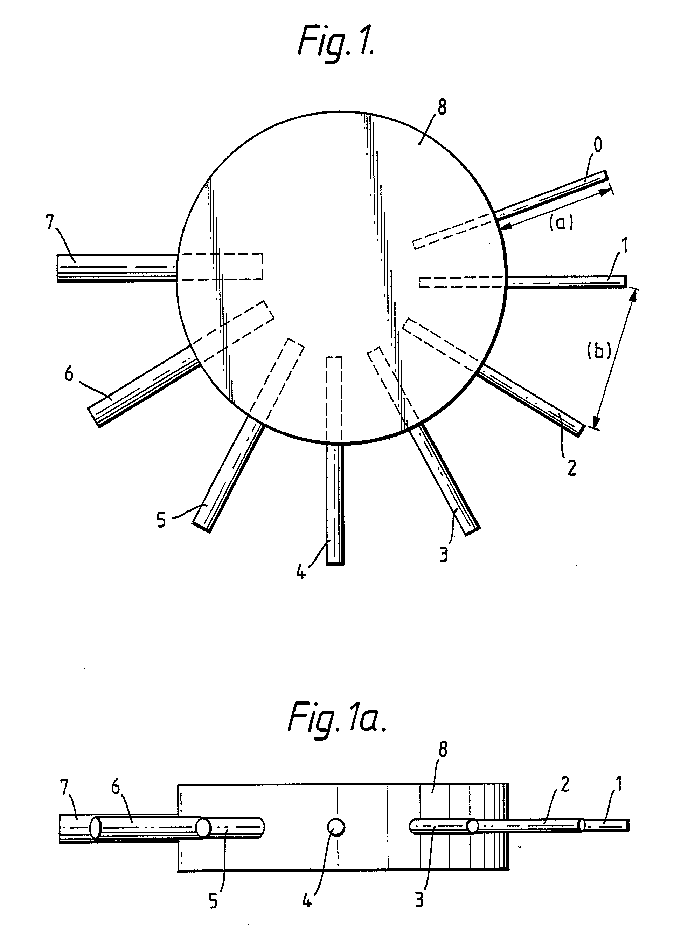

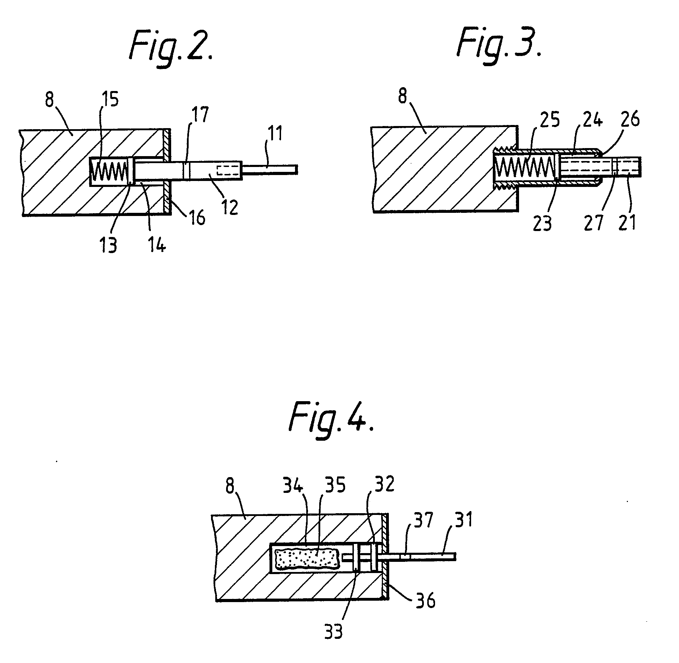

With reference to Figure 1, 7 probes labeled (0), (1), (2), (3), (4), (5), (6), (7) are embedded in a base member (8). Each probe extends outwardly from the base number for a distance (a) such that the spacing at the outer end of each probe is spaced a distance from each other (b) equivalent to at least 2 cm. While the figure shows a cylindrical base member, other configurations are operable such as rectangular, hexagonal, square, star shaped, elliptical which is sufficiently large to be hand-held. The size used for the base member is dictated by the ease of handling in the application of the method of the invention. The instrument shown in Figure 1 having the fixed probe arrangement requires a skilled technician to administer the test such that a relatively uniform pressure is administered in practice. However, an improved instrument is found in Figure 2 and Figure 3 which incorporates a means for controlling the force such that an even pressure is applied for each probe regardless of the cross-sectional size placed in contact with the skin. Such force control means comprise coil springs, leaf springs, and elasto mers such as rubbers flexible elastomer foams, and fluids.

Figure 2 provides for an arrangement wherein probe (11) is threaded into sliding rod (12) provided with flange (13). The diameter of flange (13) is adjusted to slide in well (14) within base member (8) having flange means (16) to prevent rod (12) from pulling out of the well. A constant force is applied by coil spring (15) when probe (11) is forced against the surface of the skin to move rod (12) into the well against the force of the spring to a position marked by line (17) which functions as a stop position. As the cross section of probe (11) is increased, the compressive force of spring (15) can be increased to provide a constant pressure when pushed against the surface of the skin to stop position (17).

In Figure 3 is shown an alternative arrangement wherein hollow probe (21) is slidably mounted in barrel (24) and held within the barrel provided by stop sleeve (26) and shoulder (23). In this arrangement, identical coil springs (25) can be applied for all sizes of probes wherein the cross sectional area is maintained constant by a variation in wall thickness of the hollow tubing.

Figure 4 provides an arrangement wherein probe (31) is slidably mounted in well (34) and held by retainer (32) and flange (36) and guide means (33). when probe (31) is pressed inwardly it is engaged by elastomer foam (35) which provides outward force until the probe barrel reaches marker point (37). The elastomer foam provides uniform constant pressure for larger and smaller probes.

A better understanding of the practice of the invention is gained by referring to the following nonlimiting example.

## EXAMPLE 1

The tactile circumference discriminator hand-held device of Figure 1 was used to assess tactile sensitivity as a measure of peripheral nerve dysfunction. The device consists of a central cylinder base member, made of hardened rubber, having a diameter of 60 mm and a thickness of 20 mm. Eight metal rods protrude 30 mm from the perimeter of the cylinder base member in a "sunburst" pattern. The rods are cut perpendicular to their long axis with a diameter of 1.2 mm, 1.6 mm, 2.0 mm, 2.4 mm, 2.8 mm, 3.2 mm, 3.6 mm, and 4,0.mm. Rods are arranged in an ascending size order and are sequentially labeled with 0 corresponding to the smallest and 7 corresponding to the largest rod.

Prior to testing the skin overlying the sites to be tested is cleaned. Limb temperature is monitored, using a surface electrode, and stabilized between 31-37°C for the upper limb and 30-36°C for the lower limb. Each subject is given an opportunity to become familiar with the testing device and the expected sensation. During this period the technician touches the subject with a number of rod end surfaces, selected randomly. For stimulation of the index finger, the technician supports the subject's hand, palm side up, with the technician's thumb resting across the subject's metacarpal phalangeal joint. Rods are brought into contact with the center of the dorsal surface of the index finger (IF), distal to the second inter-phalangeal joint. For stimulation of the great toe, the foot is supported distal to the ankle and the center of the "pulp" of the great toe (CT) is contacted on the inferior surface. During testing the technician brings the rods into contact with the site to be stimulated for a period of 2.0 seconds, using an estimated maximal force of about 5.0 grams (techni cians learn this by practice); rods are held perpendicular to the stimulated site. The time between repeat contacts for each trial is standardized at 3.0 seconds and the intertrial interval is held at approximately 5.0 seconds. Throughout testing, the subject is prevented from viewing the site of contact by use of an opaque screen.

At the beginning of each session the subject was given the following instructions:

"For each trial you will be touched twice -you must determine which stimulus feels bigger -pressure is not important, only size - the task will become progressively more difficult, if you are uncertain please guess."

All trials consist of two stimulus presentations with one always being the end surface of the smallest rod, (0), such that the total surface touches the skin. The order of rod presentation within a trial (i.e. largest rod first or second) is determined by a computer generated random sequence. Initially, the subject is presented with 7 vs. 0. This level is repeated. If the subject correctly identifies the larger rod end surface on both presentations the second trial is presented using the next smallest end surface. If one of the initial presentation at a particular level is incorrectly identified a third stimulus is presented at that level. If 2 out of 3 are correct the next smaller level (harder) is selected, if 2 out of 3 are incorrect the next larger level (easier) is utilized. Testing is complete when the subject has made 2 errors at a given level (not necessarily in sequence) followed by 2 consecutive correct responses at the next larger level. Threshold is the smallest size correctly identified. A typical sequence proceeds a follows:

| Probe Size | Presentations | | |
|---|---|---|---|
| | 1 | 2 | 3 |
| #7 | correct | correct | |
| #6 | correct | correct | |
| #5 | correct | correct | |
| #4 | correct | wrong | correct |
| #3 | wrong | wrong | |
| #4 | correct | correct | |
| #3 | wrong | correct | wrong |
| #4 | correct | correct | |
| Threshold Level Determined = 4 | | | |

If a subject cannot reliably distinguish between the largest and smallest rod (7 vs. 0) a Threshold Level of J7 is entered.

Results from tests conducted as described above on typical subjects selected from the population of Northeastern United States are listed in Table 1.

### Table 1

| NORMAL | | | | DIABETIC (ASYMPTOMATIC) | | | | DIABETIC (SYMPTOMATIC) | | | |
|--------|--|--|--|-------------------------|--|--|--|------------------------|--|--|--|
| Threshold Level | | | | Threshold Level | | | | Threshold Level | | | |
| Sex | Age | IF | GT | Sex | Age | IF | GT | Sex | Age | IF | GT |
| F | 42 | 2 | 2 | M | 39 | 1 | – | M | 61 | 2 | 5 |
| F | 71 | 1 | 2 | M | 40 | 3 | 7 | M | 66 | 3 | 5 |
| M | 48 | 2 | 3 | F | 40 | 2 | 3 | M | 57 | 2 | 7+ |
| F | 9 | 2 | 1 | F | 34 | 3 | 5 | F | 64 | 2 | 7+ |
| F | 30 | 3 | 2 | M | 54 | 4 | 7+ | F | 66 | 4 | 7+ |
| F | 31 | 3 | 2 | F | 33 | 1 | 2+ | M | 60 | 1 | 7+ |
| F | 33 | 2 | 1 | M | 33 | 1 | 3 | F | 61 | 4 | 7+ |
| M | 29 | 1 | 2 | F | 47 | 2 | 2 | M | 65 | 5 | 7+ |
| F | 26 | 1 | 3 | M | 20 | 2 | 1 | M | 40 | 7+ | 7+ |
| F | 28 | 2 | 3 | F | 40 | 5 | 3 | F | 51 | 2 | 7+ |
| M | 26 | 1 | 2 | F | 47 | 1 | 2 | M | 53 | 4 | 7+ |
| M | 47 | 2 | 2 | M | 54 | 2 | 7 | F | 50 | 4 | 7+ |
| F | 36 | 1 | 2 | M | 25 | 2 | 3 | | | | |
| F | 46 | 2 | 2 | F | 18 | 1 | 3 | | | | |
| M | 49 | 3 | 5 | M | 33 | 1 | 2 | | | | |
| F | 43 | 1 | 1 | | | | | | | | |
| M | 43 | 3 | 2 | | | | | | | | |
| M | 48 | 4 | 3 | | | | | | | | |
| F | 42 | 1 | 2 | | | | | | | | |
| F | 31 | 1 | 1 | MEAN AGE = 37 | | | | MEAN AGE = 58 | | | |
| F | 24 | 2 | 1 | | | | | | | | |
| F | 16 | 3 | 2 | MEAN IF = 2.07 | | | | MEAN IF = 3.33 | | | |
| F | 18 | 1 | 1 | S.D. = 1.18 | | | | S.D. = 1.60 | | | |
| F | 29 | 1 | 2 | MEAN GT = 3.57 | | | | MEAN GT = 6.67 | | | |
| F | 30 | 1 | 1 | S.D. = 1.99 | | | | S.D. = 0.75 | | | |
| F | 32 | 4 | 7+(*) | | | | | | | | |
| M | 41 | 1 | 3 | | | | | | | | |
| M | 12 | 2 | 2 | | | | | | | | |
| F | 23 | 2 | – | | | | | | | | |
| M | 37 | 1 | – | | | | | | | | |
| F | 56 | 6 | – | | | | | | | | |
| F | 45 | 1 | – | | | | | | | | |
| F | 25 | 1 | – | | | | | | | | |
| F | 34 | 2 | – | | | | | | | | |
| M | 34 | 1 | – | | | | | | | | |
| M | 41 | 2 | 4 | | | | | | | | |
| M | 49 | 3 | 6 | | | | | | | | |
| M | 13 | 2 | 1 | | | | | | | | |
| M | 13 | 1 | 1 | Sampling Sites: | | | | IF = index finger | | | |
| M | 12 | 1 | 3 | | | | | GT = great toe | | | |
| M | 12 | 1 | 1 | | | | | | | | |
| F | 64 | 4 | 5 | (*) values of >7 were entered as 7.0 for | | | | | | | |
| | | | | calculation of means | | | | | | | |

MEAN AGE = 58

MEAN IF = 1.93  
S.D. = 1.12  
MEAN GT = 2.37  
S.D. = 1.46

## Claims

1. A method for assessing human neurological function which comprises the procedure of pressing against a surface of the subject's skin a series of three or more probes having graduated cross-sectional end dimensions ranging in size from about 1 mm to about 9 mm, obtaining a discriminatory reponse from the subject with regard to determining the difference between the said end dimensions, and thereafter comparing the said response with a normal response representing no dysfunction.

2. A method as claimed in claim 1 wherein the skin surface utilised is located at a distal portion of the body such as the fingertips or toe pads.

3. A method as claimed in claim 1 or 2 wherein the probes are elongated solids rods or hollow tubes, having planar end surfaces which are pressed against the surface of the skin during the method.

4. A method as claimed in claim 1, 2 or 3 wherein said normal response representing no dysfunction represents a normal subject's ability to discriminate between graduated probe dimensional differences of 0.8-2 mm.

5. A method as claimed in any one of claims 1-4 wherein the graduated cross sectional size of said series of probes differs in increments of 0.2-0.4 mm.

6. A method as claimed in any one of claims 1-5 wherein the probes are pressed against the skin area in paired sequences according to a predetermined pattern.

7. A method as claimed in any one preceding claim which is part of the intermediate diagnosis of a human disease accompanied by neurological dysfunction.

8. A device for assessing neurological function which comprises a base member having fixed therein a series of at least three probes having graduated cross-sectional end dimensions ranging in size from about 1 mm to about 9 mm, the probes extending outwardly from the base member and terminating with a planar end surface, the end surfaces being separated from each other by a distance of at least 2 cm.

9. A device as claimed in claim 8 wherein the probes are elongated solid rods or hollow tubes.

10. A device as claimed in claim 8 or 9 wherein the probes are mounted within the base member in an arrangement permitting inward movement of a probe against a means for forcing said probe outwardly.

# Fig.1.

# Fig.1a.

## Fig.2.

8  15  17  11
13  14  16  12

## Fig.3.

8  25  24  26
23  27  21

## Fig.4.

8  34  35  32  37  31
33  36

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-3 933 148 (WYLER) <br> * Figures 1-5; column 2, line 30 - column 3, line 1 * | 1-3,7-10 | A 61 B 5/00 <br> A 61 B 5/16 <br> A 61 B 5/103 |
| A | US-A-3 074 395 (KEVORKIAN) <br> * Figurs 1-4; column 3, line 25 - column 4, line 35 * | 1-3,7-9 | |
| A | PLASTIC AND RECONSTRUCTIVE SURGERY, vol. 79, no. 5, May 1987, pages 796-801; J.H. PHILLIPS et al.: "Vibratory sensory testing in acute compartment syndromes: A clinical and experimental study" <br> * Page 796, section: "Materials and methods"; page 797, figure 1 * | 1-3,7-9 | |
| A | US-A-2 704 539 (FISHER) <br> * Figures 1-8; column 1, line 64 - column 3, line 41 * | 1-3,7-10 | |

TECHNICAL FIELDS SEARCHED (Int. Cl.5)

A 61 B

The present search report has been drawn up for all claims

| Place of search | Date of completion of the search | Examiner |
|---|---|---|
| THE HAGUE | 21-12-1989 | CHEN A.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)